# NEW EUROPEAN PATENT SPECIFICATION

(11) **EP 2 301 613 B2**
(45) Date of publication and mention of the opposition decision: **20.12.2023**
(45) Mention of the grant of the patent: 28.06.2017
(21) Application number: 10179658.9
(22) Date of filing: 24.09.2010
(51) Int. Cl.: A61M 16/00, A61B 5/02, A61B 5/00

(54) **Apparatus for assessing fluid balance status of a subject**
Verfahren, Anordnung und Vorrichtung zur Beurteilung des Flüssigkeitsgleichgewichtsstatus einer Testperson
Procédé, agencement et appareil pour évaluer l'état d'équilibre de fluides d'un sujet

(30) Priority: 29.09.2009 US 569118
(43) Date of publication of application: 30.03.2011
(73) Proprietor: General Electric Company, Schenectady, NY 12345 (US)
(72) Inventor: HUIKU, Matti, Espoo (FI)
(74) Representative: Noble, Nicholas

(56) References cited:
- EP-A1- 0 666 056
- EP-A1- 1 813 187
- EP-A1- 1 870 035
- WO-A1-2008/103389
- WO-A1-2010/111073
- GB-A- 2 459 769
- US-A1- 2001 053 881
- US-A1- 2007 032 732
- US-A1- 2010 081 942
- US-B1- 6 974 418
- SHAMIR, M. et al.: "Pulse oximetry plethysmographic waveform during changes in blood volume", British Journal of Anaesthesia, vol. 82, no. 2, 1999, pages 178-181,
- CANNESSON, M. et al.: "Pleth variability index to monitor the respiratory variations in the pulse oximeter plethysmographic waveform amplitude and predict fluid responsiveness in the operating theatre", British Journal of Anaesthesia, vol. 101, no. 2, 2008, pages 200-206,
- BENDJELID, KARIM: "The pulse oximetry plethysmographic curve revisited", Current Opinion in Critical Care, vol. 14, no. 3, 2008, pages 348-353,
- ROOKE, G. ALEC.: "Systolic pressure variation as an indicator of hypovolemia", Current Opinion in Anesthesiology, vol. 8.6, 1995, pages 511-515,

## Description

### Background of the Invention

This disclosure relates generally to the assessing of a fluid balance status of a subject, and to a procedure indicative of the fluid balance status of the subject. The subject is typically a mechanically ventilated patient, although the measurement may also be applied to spontaneously breathing patients. Below, ventilation refers generally both to mechanically ventilated subjects and to subjects without assisted ventilation.

US 6,974,418 discloses a method for calibrating an indicator. EP 0 666 056 discloses analysing changes in tidal volume.

A vast majority of hospitalized patients and all patients undergoing a surgery have an intra-venous (IV) catheter placed for delivering different types of medications and/or for infusing fluid volume and blood products, for example. Since the health and safety of the patient is often at stake, an IV infusion system must perform at the highest possible effectiveness, and the patient shall be monitored for early detection of a possible crisis situation. However, current practices in IV therapy seldom include effective crisis management with adequate monitoring and early detection of the effects of the infusions in the patient.

Effective fluid management is based on maintenance of correct fluid balance in human body. In a simple physiological model the water content of the human body is divided into three main fluid compartments: blood, interstitial body fluid (between cells), and intracellular fluid. Usually the intracellular fluid compartment is rather stable, but because of the rapid interchange of fluids that may take place between blood plasma and the interstitial compartment in sepsis and inflammatory states, for example, fluid distribution may change continuously in acute care patients. Further, in operating theatres and during post-operative care, blood and fluid loss may be considerable, which may lead, in an extreme case, to an acute need of blood transfusion and infusion of massive amounts of fluids. Excessive fluid (hypervolemia) may lead to hypertension, heart failure, pulmonary edema or electrolytic imbalance, while too little fluid (hypovolemia) may cause hypotension, hemodynamic collapse, blood centralization, or electrolyte imbalance.

Fluid management may involve infusion of crystalloid solutions, such as physiological saline, colloids or glucose solutions, depending on which compartment of body fluid is most disturbed. Crystalloids fill both the blood plasma and the interstitial compartments, while colloids fill only the plasma compartment. Glucose (small molecule) solutions are used to correct the fluid content of the whole body. In acute care, nutrition and tight glucose control are also beneficial to the patient, and there is also clinical evidence that an effective fluid management can improve patient outcome and reduce hospital costs in acute care.

A conventional procedure for evaluating the fluid balance status of a patient is a method termed fluid challenge. Fluid challenge refers to rapid administration of intra-venous fluids and is normally used to detect hypovolemia. The effect of the administration of a fluid bolus may be monitored through a cardiac output monitor, for example, or by monitoring an appropriate physiological parameter, such as central venous pressure (CVP). If there is no clear increase in the CVP in response to the administration of the fluid, the presence of hypovolemia is likely, while a clear increase in the CVP suggests the absence of hypovolemia.

A major drawback of the fluid challenge is the rather long period needed for the evaluation, since the effect of the administered fluid in the human body is slow. Furthermore, fluid challenge involves the risk of excessive fluid administration.

It is also possible to evaluate the fluid balance status automatically through cardiological data, especially blood pressure data, obtained from the patient. In this method, the blood pressure data is used to determine a patient blood volume status indicator, such as systolic blood pressure variation (SPV), the delta down component of SPV, or pulse pressure variation.

A further drawback of the above methods is that they require invasive catheterization for the administration of the fluid bolus and/or for the monitoring of the physiological parameter. This is a clear disadvantage, especially if the catheter is not needed for other clinical assessments or IV drug delivery.

### Brief Description of the Invention

The above-mentioned problems are addressed herein which will be comprehended from the following specification.

The present invention provides an apparatus as defined in claim 1.

Various other features, objects, and advantages of the invention will be made apparent to those skilled in the art from the following detailed description and accompanying drawings.

### Brief Description of the Drawings

FIG. 1 illustrates one embodiment of a system for monitoring the fluid balance status of a subject;
FIG. 2 is a flow diagram illustrating one embodiment of the method performed in the system of FIG. 1;
FIG. 3 is a block diagram illustrating one embodiment of the physiological parameter unit of FIG. 1;
FIG. 4 illustrates the fluid balance status assessment carried out in the fluid status determination unit of FIG. 1 based on change caused in a blood volume parameter;
FIG. 5 illustrates the dependency between respiration rate and depth of respiration modulation present in a plethysmographic signal; and
FIG. 6 shows an embodiment of the operation of the fluid status determination unit of FIG. 1;
FIG. 7 shows another embodiment of the operation of the fluid status determination unit of FIG. 1; and
FIG. 8 illustrates one embodiment of an apparatus for assessing the fluid balance status of a subject.

### Detailed Description of the Invention

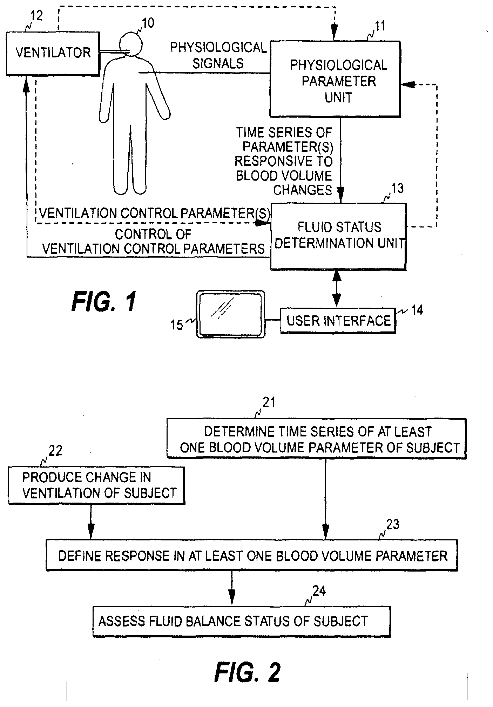

FIG. 1 illustrates an embodiment of a system for monitoring fluid balance status (volemia status) of a mechanically ventilated patient/subject 10. The system comprises a physiological parameter unit 11 for measuring successive values for at least one physiological parameter responsive to the blood volume changes in the subject. Since the parameter(s) determined in unit 11 are responsive to the blood volume changes in the subject, the said parameter(s) is/are also termed blood volume parameter(s) in this context. As discussed below, the blood volume parameter(s) may be parameters derived from photoplethysmographic data, i.e., parameters that are rather closely related to blood volume changes. Furthermore, each final blood volume parameter output from unit 11 may be an enhanced parameter derived from several different blood volume parameters or from successive values of one blood volume parameter.

The system of FIG. 1 further includes a ventilator 12 for mechanically ventilating the subject and a fluid status determination unit 13 for determining the fluid balance status of the subject. The determination unit 13 is connected to the physiological parameter unit for receiving the time series of the blood volume parameter(s) determined therein. The determination unit is further connected to the ventilator for receiving at least one ventilator control parameter from the ventilator and/or for changing at least one ventilator control parameter.

The operation of the system may be controlled through an appropriate user interface 14, which may comprise an integrated or a separate display unit 15. The fluid status determination unit 13 determines the fluid balance status of the subject based on the blood volume parameter(s) received from unit 11 and the ventilator control parameters received from the ventilator 12. The determination of the fluid balance status is based on a temporary change caused deliberately in at least one ventilator control parameter. More specifically, the fluid status determination unit 13 determines the fluid balance status based on the change detected in the blood volume parameter(s) in response to the change made in the ventilator control parameters. The user may initiate the change in the ventilator control parameters through the user interface. The new control parameters may be supplied to the ventilator either directly from the user interface or through the fluid status determination unit 13. If the adjustment of the ventilator control parameters occurs through the fluid status determination unit 13, and the said unit is thus aware of the new control parameters, it may not be necessary to supply the control parameters as feedback information from the ventilator to the fluid status determination unit. In the embodiment of FIG. 1, the ventilator and the control functionalities within the user interface and/or unit 13 thus form a control unit that may be used to produce a predetermined change in the ventilation of the subject.

The blood volume parameter(s) to be determined in the physiological parameter unit may also be controlled through the user interface, i.e., the user may select the parameter(s) to be determined from a wider selection of possible parameters.

FIG. 2 illustrates one embodiment of the steps carried out in the system of FIG. 1. A physiological signal, such as a photoplethysmographic signal, is measured from the subject and a time series of at least one blood volume parameter is determined based on the waveform data of the physiological signal in the physiological parameter unit 11 (step 21). Concurrently with the determination of the time series of the at least one blood volume parameter, a change is caused in the ventilation of the subject (step 22), thereby to modulate the blood flow of the subject and thus also the at least one blood volume parameter. The resulting response, i.e., change, in the at least one blood volume parameter is then determined in the fluid status determination unit (step 23). Based on the response, the fluid balance status of the subject and thus also the need of fluid therapy can be assessed (step 24).

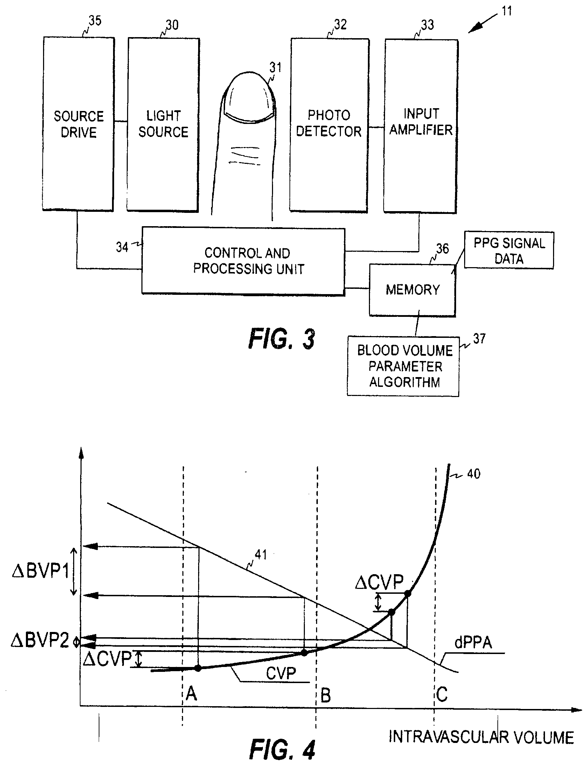

In an embodiment of the system, the physiological parameter unit 11 is based on a conventional finger/ear pulse oximeter. FIG. 3 illustrates this embodiment of the physiological parameter unit 11. A pulse oximeter normally comprises a computerized measuring unit and a probe attached to the patient, typically to a finger or ear lobe. The probe includes a light source for sending an optical signal through the tissue and a photo detector for receiving the signal transmitted through or reflected from the tissue. On the basis of the transmitted and received signals, light absorption by the tissue may be determined. During each cardiac cycle, light absorption by the tissue varies cyclically. During the diastolic phase, absorption is caused by venous blood, non-pulsating arterial blood, cells and fluids in tissue, bone, and pigments, whereas during the systolic phase there is an increase in absorption, which is caused by the inflow of arterial blood into the tissue part on which the sensor is attached. Pulse oximeters focus the measurement on this pulsating arterial blood portion by determining the difference between the peak absorption during the systolic phase and the background absorption during the diastolic phase. Pulse oximetry is thus based on the assumption that the pulsating component of the absorption is due to arterial blood only.

Traditional pulse oximeter measures absorption at two different wavelengths, i.e. the probe of a traditional pulse oximeter includes two different light emitting diodes (LEDs) or lasers, while an advanced pulse oximeter may utilize a greater number of wavelengths. The wavelength values widely used are 660 nm (red) and 940 nm (infrared), since two species of hemoglobin have substantially different absorption at these wavelengths. Each LED is illuminated in turn at a frequency which is typically several hundred Hz.

With reference to FIG. 3, light transmitted from a light source 30 including at least two LEDs or lasers passes into patient tissue, such as a finger 31. The light propagated through or reflected from the tissue is received by a photo detector 32, which converts the optical signal received at each wavelength into an electrical signal pulse train and feeds it to an input amplifier 33. The amplified signal is then supplied to a control and processing unit 34, which converts the signals into digitized format for each wavelength channel. This digitized photoplethysmographic (PPG) signal data may then be processed or stored in a memory 36 of the control and processing unit 34.

The control and processing unit further controls a source drive 35 to alternately activate the LEDs or lasers. As mentioned above, each LED or laser is typically illuminated several hundred times per second. With each LED is illuminated at such a high rate as compared to the pulse rate of the patient, the control and processing unit obtains a high number of samples at each wavelength for each cardiac cycle of the patient. The value of these samples varies according to the cardiac cycle of the patient.

In a normal pulse oximeter, the PPG signal data is processed by an SpO₂ algorithm. However, the physiological parameter unit of FIG. 1 does not necessarily utilize an SpO₂ algorithm, since for the assessment of the fluid balance status the calculation of the SpO₂ values may not be necessary. Instead, the control and processing unit 34 executes a blood volume parameter algorithm 37 to determine at least one blood volume parameter based on the PPG signal data. The resulting time series may be stored in the memory 36 or supplied directly to the fluid status determination unit 13.

In the embodiment of FIG. 3, a conventional pulse oximeter is thus employed to collect photoplethysmographic (PPG) waveform data from the subject. Based on the data, the control and processing unit of unit 11 determines a time series of one or more blood volume parameters, such as perfusion index (PI), plethysmographic pulse amplitude (PPA), delta plethysmographic pulse amplitude (dPPA), delta systolic plethysmographic amplitude peak (dPSA), delta diastolic plethysmographic amplitude (dPDA), or the respiratory variation of the plethysmographic baseline level (dBaseDC), where baseline level refers to the DC component of the plethysmographic signal, which represents the portion of tissue, venous blood, and non-pulsed arterial blood of the measured total absorption. In order to detect the respiratory plethysmographic baseline fluctuations within a ventilation cycle, it is advantageous to analyze the total light transmission signal, i.e. the composite signal (AC+DC), before calculating the plethysmographic pulse wave (AC/DC), from which the other fluid balance parameters are extracted (AC refers to the AC component of the of the plethysmographic signal, which represents the portion of the pulsed arterial blood of the measured total absorption).

To measure the dPPA, the control and processing unit extracts ventilation induced pulse variation by detecting the highest and the lowest plethysmographic pulse amplitude (PPA) over substantially one ventilation cycle. For this, unit 11 may receive ventilation phase information from the ventilator or derive the phase information from the PPG signal. The dPPA is then obtained as the difference in percentage from the average pulse height (dPPA=100*[PPAmax-PPAmin]/{[PPAmax+PPAmin]/2}). Alternatively, the highest and lowest peak or valley values may be extracted, and the peak value and valley value variations may be calculated. Generally, a high variation of PPA or PSA over a ventilation cycle, i.e. a large dPPA or dPSA, is a signature of hypovolemia. As mentioned above, the respiratory baseline variation can best be determined directly based on the composite signal. For each heart pulse the maxima and minima of the DC signal, DCpulseMAX and DCpulseMIN, are detected. The respiratory baseline variation can then be determined as dBaseDC=100*[DCpulseMAX-DCpulseMIN]/[(DCpulseMAX+DCpulseMIN)/2].

FIG. 4 illustrates the underlying principles of the fluid balance status assessment carried out in unit 13 based on a change caused in a blood volume parameter, which is dPPA in this example. FIG. 4 depicts typical behavior of central venous pressure (CVP) and dPPA in response to a fluid expansion intervention at hypovolemia (around vertical line A), at normovolemia (around vertical line B), and at hypervolemia (around vertical line C). The thick curve 40 illustrates the behavior of central venous pressure (CVP), while the thin line 41 illustrates the behavior of dPPA. CVP is used here to illustrate the pressure gradient by which the right atrium of heart is filled. Thus, CVP is a parameter which reflects the amount of blood returning to the heart and which is directly affected by the control of lung pressure. The lung pressure in turn either reduces or increases the average filling pressure of the heart. It is to be noted that CVP needs not to be measured, but is used here to illustrate that when ventilation (lung pressure) is changed, the resulting change in the blood volume parameter (dPPA) is dependent on the volemia status of the subject. Around vertical line A, CVP may have no change or a small increase (small curve slope), and in case of severe hypovolemia even a small decrease. When more fluid is added into the patient plasma volume, the hemoglobin is diluted, but also the response to fluid expansion changes: CVP around vertical line B typically show a clear increasing response.

It is generally known that arterial blood pressure changes in phase with ventilation. This phenomen is commonly termed the ventilatory swing. Generally, the air pressure in the lungs affects the amount of blood returning to the heart; high pressure impedes the blood flow towards the heart, while low pressure facilitates the said flow. Due to the ventilatory swing, the point on which the subject is on curve 40 of FIG. 4 moves slightly back and forth in phase with ventilation, i.e., the CVP, or in fact the filling pressure gradient, changes in phase with ventilation. This phenomen is now utilized to assess the fluid balance status of the subject by actively causing a temporary change in the ventilation of the subject, thereby to cause a temporary horizontal shift in FIG. 4. The shift may be carried out by temporarily changing at least one control parameter of the ventilator so that the lung pressure changes. The change in the ventilator control parameter then reflects as a change in the CVP, denoted ΔCVP in the figure. However, as can be seen from the figure, the extent of the resulting change in the blood volume parameter (ΔBVP1, ΔBVP2) depends on the fluid balance status of the subject. The extent of change in the blood volume parameter, such as dPPA, caused by a predetermined change in the ventilation control parameter may thus be utilized to assess the fluid balance status of the subject. As can be seen from the figure, in case of dPPA the change is more pronounced when the subject is hypovolemic (ΔBVP1 > ΔBVP2).

Since the change in the ventilation causes a change in the amount of blood returning towards the heart and thus also a horizontal shift in FIG. 4, the change in the ventilation emulates an infusion of a fluid bolus, which is used in fluid challenge for assessing the fluid balance status of the subject. However, as no actual bolus is needed in the procedure disclosed here, the assessment is quicker and involves no risk of excessive fluid administration. Furthermore, a change in the ventilation may easily be used to accomplish a shift in both horizontal directions of FIG. 4, whereas fluid challenge always results in a shift towards hypervolemia.

As indicated above, the blood volume parameter(s) generated in unit 11 is/are responsive to changes in the blood volume of the subject. In the above embodiment, the physiological parameter unit is based on a conventional pulse oximeter and the parameter(s) derived therein may include one or more of the PI, PPA, dPPA, dPSA, dPDA, and dBaseDC. However, the physiological parameter unit may also determine other blood volume parameters that are not based on plethysmographic data, such as parameters derived from heart rate, pulse rate, blood pressure, blood pulse pressure. The use of such parameters is discussed below.

In the embodiments determining SPV, the operation of unit 11 may be such that the peak values of the blood pressure in consecutive heart beats are detected first. Next, the maximum and minimum values of the peak (systolic) pressures are detected over one ventilation cycle and the difference between the minimum and maximum values is calculated. In a mechanically ventilated patient, the difference is typically calculated in two segments: the delta down (dDown) and the delta up (dUp) that sum up to the total difference. Delta down is the systolic pressure change between the minimum of the systolic pressure values and a reference systolic pressure, such as the average pressure measured during a short apnea period, while the delta up is the change between the maximum value and the said reference value. In normal volemia status (normovolemia), SPV is typically 8-10 mmHg, while the down and up components thereof, dDown and dUp, are typically 5-6 mmHg and 2-4 mmHg, respectively.

Higher SPV and dDown are responsive to blood volume changes and good indicators of hypovolemia in a mechanically ventilated patient, in which dDown can be as much as 20 mmHg and is mainly responsible of the total pressure variation. Correspondingly, low values are indicative of hypervolemia.

Another possible blood volume parameter that may be determined in unit 11 is the delta arterial Pulse Pressure (dPP) that reflects pulse pressure variability over one ventilation cycle. The value of dPP may be measured in percents, dPP=100*[PPmax-PPmin]/{[PPmax+PPmin]/2}, where PPmax is the maximum and PPmin the minimum of the pulse pressure values in one ventilation cycle, and each pulse pressure value represents the difference of the systolic and diastolic pressures in one heart beat. Low values of dPP are indicative of hypervolemia and high values of hypovolemia.

Some other blood volume parameters that may be determined in the physiological parameter unit 11 are summarized in Table 1 below. The first four rows involve cardiovascular parameters, i.e., parameters that depend on the operation of the cardiovascular system and are thus rather closely related to blood volume changes, while the last three rows show other parameters reflecting changes in blood volume.

**Table 1:**

| | |
|---|---|
| Blood circulation and peripheral perfusion, and ventilatory blood circulation variation | PI, PPA, dPPA, dPSA, dPDA, dBaseDC. |
| Blood pressure and blood pressure variation | SPV, dDown, dPP, CVP, Pulmonary artery occlusion pressure (PAOP), Pulmonary artery wedge pressure (PWP), systolic Blood Pressure (sysBP), Mean Arterial Pressure (MAP) |
| Heart rate variation | Respiratory sinus arrhythmia (RSA) |
| Cardiac ejection volume | Cardiac output (CO), cardiac index (CI), stroke index (SI) |
| Temperature | Finger/toe temperature, core-peripheral temperature gradient |
| Exhaled gas concentrations, venous oxygen saturation | Venous saturation (SvO₂), End tidal carbon dioxide (EtCO₂), End tidal oxygen concentration (EtO₂) |
| Urine secretion | Urine flow, bladder volume |

In one embodiment, the ventilation control parameter to be controlled is the positive end-expiratory pressure (PEEP) that refers to the use of an elevated pressure during the expiratory phase of the ventilatory cycle. The control of the value of this elevated pressure changes the amount of blood returning to the heart, and the control of PEEP may thus be used for accomplishing a blood volume parameter change that depends on the volemic status of the subject. The control of PEEP may be used in association with a blood volume parameter derived from PPG data, such as dPPA. Other ventilator control parameters that may be used are the ventilation rate, the tidal volume, or the ventilation airway pressure pattern (ventilation mode). The control of the ventilation control parameters may be done such that normoventilation is maintained all the time (end-tidal CO₂ is kept at about 5%). If the ventilation rate is the parameter to be changed, the tidal volume is altered to keep the same minute volume.

The length of the temporary change of the ventilation control parameters may vary from one to several ventilation cycles, for example. Typically, the ventilator control parameters may be returned to their previous values after a duration of about 30 seconds. However, the new settings may also be maintained permanently.

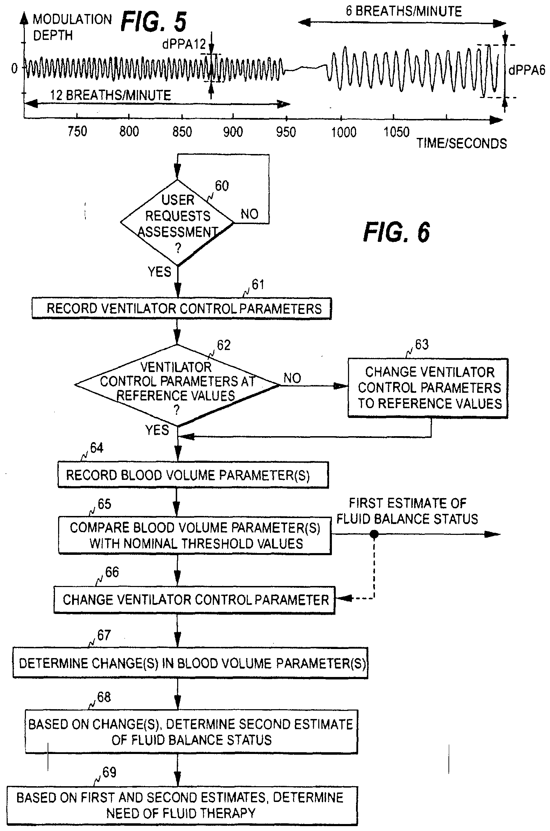

The depth of the respiratory modulation in the plethysmographic pulse wave amplitude and baseline level has larger inter- and intrapatient variability than what is observed in the corresponding blood pressure parameters. In addition to the lung pressure directly modulating the blood return to the heart, the sympathetic regulation of the autonomic nervous system affects the peripheral pulse wave at the measurement site. At certain respiration rates, corresponding typically to a 10 second respiration interval, the autonomic amplification of the respiratory modulation depth is significant and must be taken into account when interpreting the pulse wave changes. FIG. 5 illustrates the dependency on the respiration rate of the depth of the respiratory modulation in a plethysmographic pulse wave amplitude. The figure shows an example of the depth of the respiration modulation in a plethysmographic signal at respiration rates of 12 and 6 breaths per minute. About at t=950 sec, the respiration rate is changed from 12 to 6 breaths per minute. As can be seen from the figure, the respiratory modulation depth at 6 breaths per minutes, dPPA6, is about two times larger than the modulation depth at the higher rate of 12 breaths per minute, dPPA12. As the fluid balance status of the patient is unchanged in the example of FIG. 5, the dPPA value cannot directly be used as a reliable blood volume parameter. The autonomic amplification also depends on the fluid status of the patient itself. In normo- and hypervolemia the effect is smaller than in hypovolemia. Therefore, in one embodiment, a change of ventilation rate is made in the ventilator to calibrate the blood volume parameter value in reference to a predetermined respiration rate. The calibration is needed particularly for plethysmographic peripheral waveform parameters, but may also be used for other blood volume parameters..

FIG. 6 illustrates an embodiment of the operation of the fluid status determination unit 13. The fluid status determination unit 13 receives the blood volume parameter, such as dPPA, from the physiological parameter unit 11, and the ventilator control parameters from the ventilator 12 or from the user interface 14. The operation of the fluid status determination unit 13 is now described regarding a mode in which the fluid status determination unit automatically controls the ventilator. It is further assumed here that the blood volume parameter is dPPA provided from a SpO₂ measurement unit.

The user initiates the fluid balance determination via the user interface unit 14 by requesting an automated artificial fluid challenge (step 60/yes). The fluid status determination unit 13 then records the current ventilator control parameters (step 61) and compares the obtained values with predetermined reference values (nominal values) under which the dPPA threshold values for hypovolemia, normovolemia and hypervolemia are pre-set (step 62). If the ventilator control parameters are at their reference values, the blood volume parameter(s) is/are recorded (step 64). However, if this is not the case, the ventilator control parameters are first controlled to their reference values (step 63), and the blood volume parameter(s) is/are then recorded after a certain time period, such as 30 seconds, needed for stable readings (step 64). A first estimate of fluid balance status may then be obtained by comparing the blood volume parameter with its predetermined threshold values set for each volemic state (step 65). For instance, at respiration rate of 12 breaths per minute and a tidal volume of 500 ml with inspiratory-expiratory ratio I:E of 1:2, the hypovolemic, normovolemic and hypervolemic threshold values of dPPA are, respectively, larger than 10%, between 2 and 10 % and smaller than 2 % of the plethysmographic pulse amplitude PPA.

After this, one of the ventilator control parameters, preferably PEEP or respiration rate, is changed at step 66 and the resulting change in the blood volume parameter, i.e., dPPA, is determined after a certain time interval (step 67). A second estimate of the fluid status of the patient is now obtained by comparing the change in the dPPA to predetermined ranges for hypo-, normo- and hypervolemia (step 68). For instance, if PEEP is altered from zero to 2 cmH₂O, hypovolemia is detected if the dPPA change is more than +100% from the pre-change value.

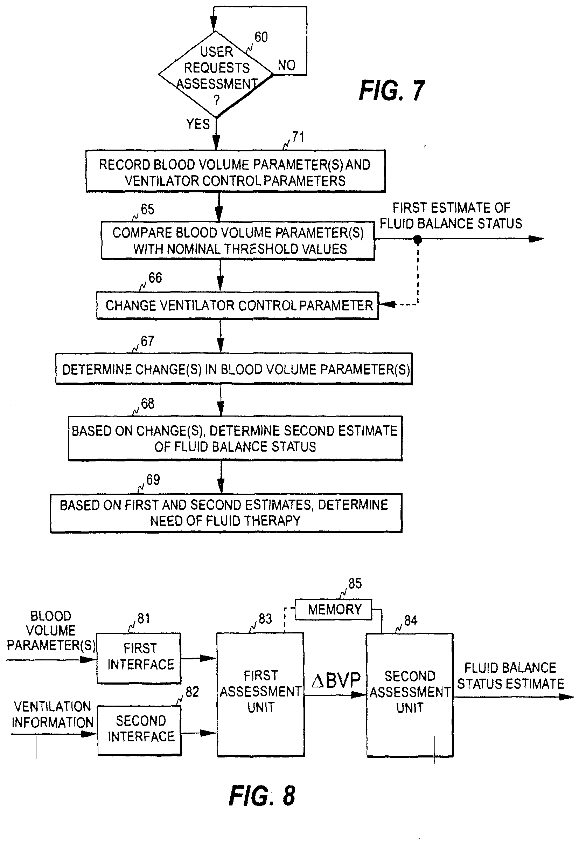

In another embodiment, which is illustrated in FIG. 7, the first estimate of the fluid status is obtained by using a pre-calculated relationship of the ventilator control parameters and the blood volume parameter, such as dPPA, thereby omitting the change of the ventilator control parameters to their reference values. The relationship can be stored, for example, in a look-up table that maps the dPPA values to respiration rate, tidal volume, and other ventilator control parameters, and to the threshold values of the three volemic states. Consequently, in this embodiment the values of the blood volume parameter and the ventilator control parameters are recorded immediately (step 71), without first changing the ventilator control parameters to predetermined reference values. Furthermore, in step 65 the threshold values of the blood volume parameter are retrieved from the look-up table based on the current values of the blood volume parameter and the ventilator control parameters, thereby to enable the comparison and to obtain the first estimate of the fluid balance status. The calibration needed particularly for plethysmographic peripheral waveform parameters may in the embodiment of FIG. 7 be implemented into the look-up table by storing calibrated values corresponding to different ventilation patterns. Otherwise the embodiment of FIG. 7 corresponds to that of FIG. 6.

The first and second estimates of the fluid balance status, which are respectively produced from the dPPA values and the dPPA change values obtained in steps 65 and 68, respectively, may take the form of user messages that indicate the dPPA value or change value and the nearest volemic state threshold value. In step 69, the fluid status determination unit may use a set of rules for determining a combined estimate of the fluid balance status and the need of fluid therapy. The fluid status determination unit 13 may thus display the first and second fluid status estimates and/or a combined fluid status estimate on the display unit 15. After this, the fluid status determination unit may restore the original control parameter settings of the ventilator. The results of the automated fluid assessment procedure may be trended, stored in the memory of the monitor, and/or sent to a hospital information system network, for example. The first and second estimates may also take the form of any appropriate graphical presentation, such as a bar in which the position of the subject is indicated with respect to one or more volemic thresholds.

In the embodiments of FIG. 6 and 7 the first estimate is obtained based on the blood volume parameter value and the second estimate based on the change in the blood volume parameter. However, the generation of the first estimate may be omitted. This is the case in the embodiment of FIG. 2, where only one estimate, i.e., indicator of fluid balance status, is obtained based on the change in the blood volume parameter in response to a controlled change in the ventilation. In one embodiment, the change in the blood volume parameter may be used directly as the indicator of fluid balance status presented to the user, and the user may make the decisions regarding the fluid balance status based on the said change. In case of two estimates, more weight may be put on the second estimate that is based on the change in the blood volume parameter. Furthermore, in case of two estimates the first estimate may be used to define the ventilation change to be carried out subsequently in step 66. This is illustrated with dashed arrows in FIGs. 6 and 7. For example, if the first estimate is indicative of hypovolemia, a lower PEEP value may be used than if the first estimate is indicative of hypervolemia. The use of the first estimate for adjusting the ventilation change to be carried out improves the sensitivity of the assessment.

The fluid status determination unit 13 and the physiological parameter unit 11 may continue the above operation and inform about the changes of the parameters on the display. In one embodiment, the fluid status determination unit 13 may suggest a new artificial fluid challenge by sending a query to the user. After user acceptance, the above-described automated fluid challenge procedure may be repeated. The procedure may also be repeated at regular time intervals, such as every 30 minutes.

The above assessment procedures may also be applied to spontaneously breathing subjects. In this case the subject may change his or her ventilation pattern by a request of a physician. For instance, the subject may first breath normally and then take a deep breath after which he or she may return to normal breathing. The physiological parameter unit 11 monitors the blood volume parameter(s) and the fluid status determination unit 13 assesses the need of fluid therapy based on the changes caused the deep breath in the blood volume parameters. The method may further be improved by simultaneously controlling the position of the subject. The subject may first breath normally at a supine position, then the subject is tilted on a tilt table by for instance 10 degrees, which results in a change in the blood volume return to heart. The change in the blood volume parameter(s) between the supine and tilted positions is determined. A respiration pattern change, such as a deep breath, may be made at both positions.

A monitor assessing the fluid balance of a subject does not necessarily comprise all the elements shown in FIG. 1, but the monitor may receive the blood volume parameters from an external device. Furthermore, the monitor may not control the ventilator, but may only detect a change that is made in the ventilation of the subject. Therefore, a simplified embodiment of the monitor comprises the elements shown in FIG. 8. A first interface unit 81 is configured to receive successive values of the blood volume parameter(s) from an external unit, while a second interface unit 82 is configured to receive ventilation information and to detect, based on the received information, a change in the ventilation of the subject. A first assessment unit 83 is configured to monitor a response that the detected change causes in at least one blood volume parameter, thereby to obtain at least one measure of change (ΔBVP). A second assessment unit 84 is then configured to assess the fluid balance status of the subject based on the at least one measure of change. As discussed above, the apparatus may comprise a memory 85, such as a look-up table, that holds threshold values for the different volemic states. The threshold values may be stored for a wide variety of ventilation patterns, especially if the ventilator is not controlled to the reference operating state prior to the ventilation change, and the memory may hold the threshold values of the volemic states for the blood volume parameter change values only or for both the blood volume parameter values and the blood volume parameter change values. That is, the apparatus may define the second estimate of the fluid balance status only or both the first and second estimates of the fluid balance status. In further embodiments of the apparatus, the fluid status determination unit of FIG. 8 may be combined with any one or more of the elements shown in FIGs. 1 and 3.

## Claims

1. An apparatus for assessing fluid balance status of a spontaneously breathing subject (10), the apparatus comprising:
- a first interface unit (81) configured to receive successive values of at least one physiological parameter responsive to blood volume changes in the spontaneously breathing subject (10),
**characterized in that** the apparatus further comprises
- a second interface unit (82) configured to detect a change in ventilation of the spontaneously breathing subject (10);
- a first assessment unit (83) configured to monitor a response that the change causes in at least one of the at least one physiological parameter, thereby to derive at least one measure of change from the at least one physiological parameter; and
- a second assessment unit (84) configured to determine the fluid balance status of the spontaneously breathing subject (10) based on the at least one measure of change.

2. The apparatus according to claim 1, wherein the first interface unit (81) is configured to receive the successive values of the at least one physiological parameter, in which the at least one physiological parameter is at least one of perfusion index (PI), plethysmographic pulse amplitude (PPA), delta plethysmographic pulse amplitude (dPPA), delta systolic plethysmographic amplitude peak (dPSA), delta diastolic plethysmographic amplitude (dPDA), and the respiratory variation of the plethysmographic baseline level (dBaseDC).

3. The apparatus according to any of the preceding claims, wherein the second assessment unit (84) is configured to compare the at least one measure of change with predetermined threshold values of different volemic states.

4. The apparatus according to claim 3, wherein the apparatus is provided with a memory (85) storing the predetermined threshold values, and wherein the predetermined threshold values include threshold values for a plurality of ventilation patterns.

## Patentansprüche

1. Vorrichtung zum Beurteilen eines Fluidausgleichsstatus einer spontan atmenden Person (10), die Vorrichtung umfassend:
- eine erste Grenzflächeneinheit (81), die zum Empfangen von aufeinanderfolgenden Werten von zumindest einem physiologischen Parameter konfiguriert ist, der auf Blutvolumenänderungen in der spontan atmenden Person (10) reagiert,
**dadurch gekennzeichnet, dass** die Vorrichtung ferner folgendes umfasst:
- eine zweite Grenzflächeneinheit (82), die zum Erkennen einer Änderung der Atmung der spontan atmenden Person (10) konfiguriert ist;
- eine erste Beurteilungseinheit (83), die zum Überwachen einer Reaktion konfiguriert ist, die die Änderung bei zumindest einem des zumindest einen physiologischen Parameters bewirkt, um dadurch zumindest eine Änderungsmessung von dem zumindest einen physiologischen Parameter abzuleiten; und
- eine zweite Beurteilungseinheit (84), die zum Bestimmen des Fluidausgleichsstatus der spontan atmenden Person (10) auf Grundlage der zumindest einen Änderungsmessung konfiguriert ist.

2. Vorrichtung nach Anspruch 1, wobei die erste Grenzflächeneinheit (81) zum Empfangen der aufeinanderfolgenden Werte des zumindest einen physiologischen Parameters konfiguriert ist, wobei der zumindest eine physiologische Parameter eines von Perfusionsindex (PI), plethysmographischer Pulsamplitude (PPA), plethysmographischer Delta-Pulsamplitude (dPPA), systolischer plethysmographischer Delat-Amplitudenspitze (dPSA), diastolischer plethysmographischer Delta-Amplitude (dPDA) und der Atmungsvariation des plethysmographischen Grundlinienpegels (dBaseDC) ist.

3. Vorrichtung nach einem der vorhergehenden Ansprüche, wobei die zweite Beurteilungseinheit (84) zum Vergleichen der zumindest einen Änderungsmessung mit vorgegebenen Schwellenwerten von verschiedenen Volumenzuständen konfiguriert ist.

4. Vorrichtung nach Anspruch 3, wobei die Vorrichtung mit einem Speicher (85) versehen ist, der die vorgegebenen Schwellenwerte speichert, und wobei die vorgegebenen Schwellenwerte Schwellenwerte für mehrere Beatmungsmuster beinhalten.

## Revendications

1. Appareil d'évaluation de l'état d'équilibre hydrique d'un sujet respirant spontanément (10), l'appareil comprenant:
- une première unité d'interface (81) configurée pour recevoir des valeurs successives d'au moins un paramètre physiologique sensible aux changements de volume sanguin dans le sujet respirant spontanément (10),
**caractérisé en ce que** l'appareil comprend en outre :
- une seconde unité d'interface (82) configurée pour détecter un changement dans la ventilation du sujet respirant spontanément (10) ;
- une première unité d'évaluation (83) configurée pour surveiller une réponse que le changement provoque dans au moins un de l'au moins un paramètre physiologique, pour ainsi déduire au moins une mesure de changement à partir de l'au moins un paramètre physiologique ; et
- une seconde unité d'évaluation (84) configurée pour déterminer l'état d'équilibre hydrique du sujet respirant spontanément (10) sur la base de l'au moins une mesure de changement.

2. Appareil selon la revendication 1, dans lequel la première unité d'interface (81) est configurée pour recevoir les valeurs successives de l'au moins un paramètre physiologique, dans lequel l'au moins un paramètre physiologique est au moins l'un parmi un indice de perfusion (PI), une amplitude de pouls pléthysmographique (PPA), une amplitude de pouls pléthysmographique delta (PPAd), un pic d'amplitude pléthysmographique systolique delta (PSAd), une amplitude pléthysmographique diastolique delta (PDAd) et la variation respiratoire du niveau initial de base pléthysmographique (dBaseDC).

3. Appareil selon l'une quelconque des revendications précédentes, dans lequel la seconde unité d'évaluation (84) est configurée pour comparer l'au moins une mesure de changement avec des valeurs de seuil prédéterminées de différents états volumiques.

4. Appareil selon la revendication 3, l'appareil étant pourvu d'une mémoire (85) stockant les valeurs de seuil prédéterminées et dans lequel les valeurs de seuil prédéterminées comprennent des valeurs de seuil pour une pluralité de modèles de ventilation.
